# FASCICULE DE BREVET EUROPEEN

(11) **EP 1 006 154 B1**
(45) Date de publication et mention de la délivrance du brevet: **17.04.2002**
(21) Numéro de dépôt: 99402629.2
(22) Date de dépôt: 22.10.1999
(51) Int. Cl.: C09B 1/20, A61K 7/13

(54) **Aminoanthraquinones cationiques, leur utilisation pour la teinture des fibres kératiniques, compositions tinctoriales les renfermant et procédés de teinture**
Kationische Aminoanthrachinone, deren Verwendung zum Färben keratinischer Fasern, diese enthaltende Färbemittel sowie Färbeverfahren
Cationic aminoanthraquinones, use thereof for the dyeing of keratinous fibres, dyeing compositions containing them and dyeing processes

(30) Priorité: 30.11.1998 FR 9815046
(43) Date de publication de la demande: 07.06.2000
(73) Titulaire: L'OREAL, 75008 Paris (FR)
(72) Inventeur: Genet, Alain, 93600 Aulnay Sous Bois (FR); Lagrange, Alain, 77700 Coupvray (FR)
(74) Mandataire: Miszputen, Laurent

(56) Documents cités:
- EP-A- 0 852 136
- FR-A- 1 379 649
- FR-A- 1 401 163
- GB-A- 1 205 365
- US-A- 5 520 707

## Description

La présente invention concerne des aminoanthraquinones comportant au moins un groupement cationique choisi parmi des chaînes aliphatiques comportant au moins une charge cationique délocalisée sur un cycle polyazoté insaturé à 5 chaînons, leur utilisation à titre de colorant direct dans les applications de teinture des matières kératiniques, en particulier des fibres kératiniques humaines, et notamment les cheveux, et plus particulièrement les compositions de teinture les renfermant.

Il est connu de teindre les fibres kératiniques, et en particulier les cheveux, avec des compositions tinctoriales contenant des colorants directs, c'est-à-dire des molécules colorantes ayant une affinité pour lesdites fibres. Le procédé de teinture qui les met en oeuvre est un procédé dit de coloration directe qui consiste à laisser pauser les colorants directs sur les fibres, puis à les rincer.

Les colorations qui en résultent sont des colorations temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.
Parmi les colorants directs connus, on a déjà décrit des aminoanthraquinones cationiques dont la charge est localisée sur l'atome d'azote. De telles aminoanthraquinones sont par exemple décrites dans les brevets français N°- 1 422 016 et son addition 87.902, 1 391 675, 1 401 163, 379 649, 1 430 089, 1 584 965, 2 050 397, 2 548 895, les brevets américains N°- 5 169 403, 5 314 505, 5 486 629, 5 520 707, et les brevets européens N°- 818 193 et 852 136.

Cependant, en teinture capillaire, on recherche en permanence des colorants directs qui présentent des caractéristiques toujours plus performantes.

C'est donc après d'importantes recherches menées sur la question que la demanderesse vient maintenant de découvrir de nouvelles aminoanthraquinones cationiques dont au moins une charge cationique est délocalisée sur un hétérocycle polyazoté insaturé à cinq chaînons et comportant donc au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé.
Cette nouvelle famille de colorants présente la particularité très avantageuse d'une plus grande solubilité dans les milieux de teinture et ces nouveaux colorants engendrent des teintures, par coloration directe, dotées d'une puissance et d'une résistance (aux diverses agressions que peuvent subir les cheveux : lumière, intempéries, shampooings, transpiration), notablement améliorée par rapport à celle des teintures réalisées avec des aminoanthraquinones cationiques connues de l'art antérieur.

Cette découverte est à la base de la présente invention.

La présente invention a ainsi pour objet les aminoanthraquinones de formule (I) suivante : 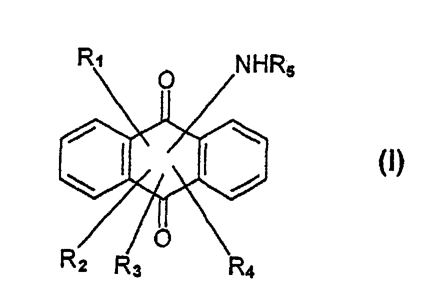 formule dans laquelle
- **R**_{**1**}**, R**_{**2**}**, R**_{**3**} et **R**_{**4**}**,** qui peuvent être identiques ou différents, représentent un atome d'hydrogène ; un atome d'halogène ; un groupement Z défini ci-après ; un radical alkyl(C₁-C₆) ; un radical monohydroxyalkyle en C₁-C₆ ; un radical polyhydroxyalkyle en C₂-C₆ ; un radical cyano ; un radical nitro ; un radical carboxy ; un radical carbamyle ; un radical sulfo ; un radical amino substitué ou non substitué, ayant les mêmes significations que NHR₅ défini ci-dessous, et pouvant être identique ou différent ; un groupement OR₆ ou SR₆ défini ci-après.
- **R**_{**5**} désigne un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'aminé est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z (définis ci-après), ou pouvant former ensemble, avec l'atome d'azote auquel ils sont rattachés, un cycle à 5 ou 6 chaînons, carboné, ou contenant un ou plusieurs hétéroatomes;
- **R**_{**6**} désigne un atome d'hydrogène ; un radical alkyle en C₁-C₆; un radical monohydroxy-alkyle en C₁-C₆ ; un radical polyhydroxyalkyle en C₂-C₆ ; un groupement Z (défini c-après) ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆; un radical trifluoroalkyle en C₁-C₆ ; un radical aminosulfonylalkyle en C₁-C₆ ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle en C₁-C₆ ; un radical alkyl(C₁-C₆)sulfinyl-alkyle en C₁-C₆ ; un radical alkyl(C₁-C₆)sulfonylalkyle en C₁-C₆ ; un radical alkyl(C₁-C₆)-carbonylalkyle en C₁-C₆ ; un radical aminoalkyle en (C₁-C₆) dont l'alkyle est substitué ou non substitué par un ou plusieurs radicaux hydroxy ; un radical aminoalkyle en (C₁-C₆) dont l'alkyle est substitué ou non substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z (définis ci-après), ou pouvant former ensemble, avec l'atome d'azote auquel ils sont rattachés, un cycle à 5 ou 6 chaînons, carboné ou contenant un ou plusieurs hétéroatomes ;
- **Z** est choisi parmi les groupements cationiques insaturés de formules (II) et (III) suivantes, et les groupements cationiques saturés de formule (IV) suivante : 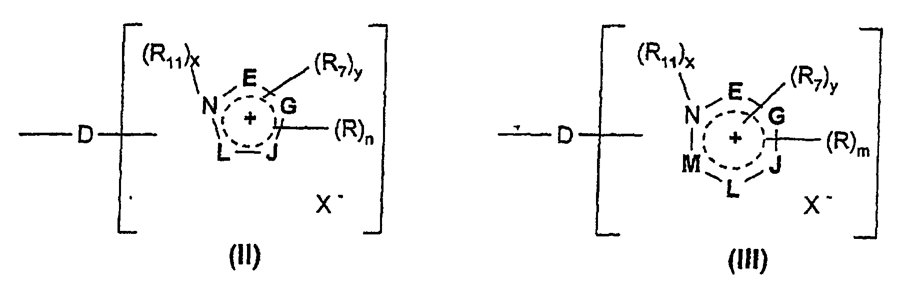 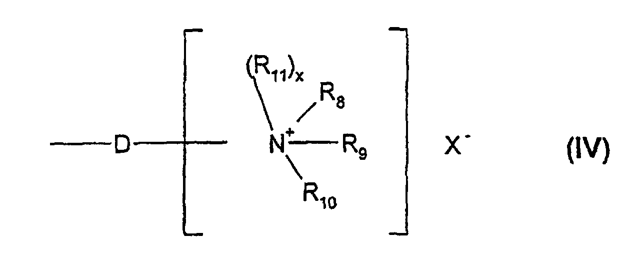 dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R'", identiques ou différents, représentent un radical alkyle en C₁-C₆, un radical monohydroxyalkyle en C₁-C₆ ou un radical polyhydroxyalkyle en C₂-C₆ ;
   - R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
   - R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'aminé est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
      l'un des radicaux R₈, R₉ et R₁₀ peut également représenter un second groupement Z, identique ou différent du premier groupement Z ;
   - R₁₁ représente un radical alkyle en C₁-C₈ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
   - x et y sont des nombres entiers égaux à 0 ou 1 ; avec les conditions suivantes :
      - dans les groupements cationiques insaturés de formule (II) :
         - lorsque x = 0, le bras de liaison D est rattaché à l'atome d'azote,
         - lorsque x = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₇ est porté par l'atome d'azote du cycle insaturé ; ou bien
            2) lorsqu'au moins un des sommets E, G, J et L représente un atome d'azote sur lequel le radical R₇ est fixé ;
      - dans les groupements cationiques insaturés de formule (III):
         - lorsque x = 0, le bras de liaison D est rattaché à l'atome d'azote,
         - lorsque x = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₇ est porté par l'atome d'azote du cycle insaturé ;
      - dans les groupements cationiques de formule (IV) :
         - lorsque x = 0, alors le bras de liaison D est rattaché à l'atome d'azote portant les radicaux R₈ à R₁₀,
         - lorsque x = 1, alors deux des radicaux R₈ à R₁₀ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
   - **X**^{**-**} représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu que :**
le nombre de groupements cationiques insaturés Z de formule (II), dans lesquels au moins un des sommets E, G, J et L représente un atome d'azote, est au moins égal à 1.

Les composés de formule (I) peuvent être éventuellement salifiés par des acides minéraux forts tels que HCI, HBr, H₂SO₄, ou des acides organiques tels qu'acétique, lactique, tartrique, citrique ou succinique.

Les alkyles et alkoxy cités ci-avant dans les formules (I), (II) (III) et (IV) peuvent être linéaires ou ramifiés.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Des composés de formule (I) sont notamment choisis dans le groupe formé par :
- le bromure de 1-[2-(9,10-dioxo-9,10-dihydro-anthracen-1-ylamino)-éthyl]-3-méthyl-3H-imidazol-1-ium,
- le sulfate acide de 1-méthyl-3-[3-(4-méthylamino-9,10-dioxo-9,10-dihydro-anthracen-1-ylamino)-propyl]-3H-imidazol-1-ium,
- le bromure de 1-[2-(4-hydroxy-9,10-dioxo-9,10-dihydro-anthracen-1-ylamino)-ethyl]-3-methyl-3H-imidazol-1-ium,
- le méthosulfate de 1-[3-(9,10-dioxo-9,10-dihydro-anthracen-1-ylamino)-propyl]-3-methyl-3H-imidazol-1-ium,
- le méthosulfate de 1-[3-(4-hydroxy-9,10-dioxo-9,10-dihydro-anthracen-1-ylamino)-propyl]-3-methyl-3H-imidazol-1-ium,
- le méthosulfate de 1-{3-[4-(2-hydroxy-ethylamino)-9,10-dioxo-9,10-dihydro-anthracen-1-ylamino]-propyl}-3-methyl-3H-imidazol-1-ium,
- le méthosulfate de 1-{3-[4-(2,3-dihydroxy-propylamino)-9,10-dioxo-9,10-dihydro-anthracen-1-ylamino]-propyl}-3-methyl-3 H-imidazol-1-ium,
- le diméthosulfate de 1,4-Bis-[3-(9,10-dioxo-9,10-dihydro-anthracen-1,4-diylamino)-propyl]-3-methyl-3H-imidazol-1-ium,
- le bromure de 1-[2-(9,10-dioxo-9,10-dihydro-anthracen-2-ylamino)-ethyl]-3-methyl-3H-imidazol-1-ium,
- le bromure de 1-[2-(9,10-dioxo-9,10-dihydro-anthracen-2-ylamino)-ethyl]-2-methyl-3H-pyrazol-1-ium,
- le bromure de 1-[2-(9,10-dioxo-9,10-dihydro-anthracen-1-ylamino)-ethyl]-2-methyl-3H-pyrazol-1-ium,
- le diméthosulfate de 1,5-bis-[3-(9,10-dioxo-9,10-dihydro-anthracen-1,5-diylamino)-propyl]-3-methyl-3H-imidazol-1-ium,
- le diméthosulfate de 1,8-bis-[3-(9,10-dioxo-9,10-dihydro-anthracen-1,8-diylamino)-propyl]-3-methyl-3H-imidazol-1-ium,
- le bromure de 1-[2-(5,8-diamino-9,10-dioxo-9,10-dihydro-anthracen-1-ylamino)-ethyl]-3-methyl-3H-imidazol-1-ium,
- le méthosulfate de 1-[3-(5,8-diamino-9,10-dioxo-9,10-dihydro-anthracen-1-ylamino)-propyl]-3-methyl-3H-imidazol-1-ium.

Les composés de formule (I) conformes à l'invention peuvent être facilement obtenus, selon des méthodes bien connues de l'état de la technique pour l'obtention des aminés quaternisées, par exemple :
- en un temps, par condensation d'une anthraquinone comportant un radical halogénoalkyle avec un composé porteur d'un radical amine tertiaire, ou par condensation d'une anthraquinone comportant un radical amine tertiaire avec un composé porteur d'un radical halogénoalkyle ;
- ou en deux temps, par condensation d'une anthraquinone comportant un radical halogénoalkyle avec un composé porteur d'une amine secondaire, ou par condensation d'une anthraquinone halogénée ou hydroxylée avec une amino(di-substituée)alkyleamine, suivie d'une quatemisation avec un agent alkylant.

L'étape de quaternisation est, généralement par commodité, la dernière étape de la synthèse, mais peut intervenir plus tôt dans la suite des réactions conduisant à la préparation des composés de formule (I).

L'invention a aussi pour objet une composition de teinture pour matières kératiniques, comprenant, dans un milieu approprié pour la teinture, une quantité efficace d'au moins une aminoanthraquinone cationique de formule (I) définie ci-avant.

L'invention a également pour objet une composition de teinture directe pour fibres kératiniques humaines, et notamment les cheveux, comprenant dans un milieu approprié pbur la teinture, une quantité efficace d'au moins une aminoanthraquinone cationique telle que définie ci-avant par la formule (I).

L'invention a pour autre objet l'utilisation des aminoanthraquinones cationiques de formule (I), à titre de colorants directs, dans des, ou pour la préparation de, compositions tinctoriales pour matières kératiniques, en particulier pour fibres kératiniques humaines telles que les cheveux.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

La concentration en aminoanthraquinone cationique de formule (I) est de préférence comprise entre environ 0,005 et 12%, et encore plus préférentiellement entre environ 0,05 et 6% en poids, par rapport au poids total de la composition tinctoriale.

La composition tinctoriale selon l'invention peut encore contenir, pour obtenir des teintes variées, outre les aminoanthraquinones cationiques de formule (I), d'autre(s) colorant(s) direct(s) classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques autres que ceux de formule (I), des colorants mono- ou di-azoïques, triarylméthaniques, aziniques, acridiniques et xanthéniques, ou encore des colorants métallifères. La proportion de tous ces autres colorants directs d'addition peut varier entre environ 0,05 et 10% en poids par rapport au poids total de la composition tinctoriale.

Les aminoanthraquinones cationiques de formule (I) peuvent également être incorporées dans des compositions tinctoriales pour la coloration d'oxydation qui contiennent des bases d'oxydation et éventuellement des coupleurs, pour enrichir en reflets les nuances obtenues avec les colorants d'oxydation.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Les solvants peuvent être présents dans des proportions de préférence comprises entre 1 et 40% en poids par rapport au poids total de la composition tinctoriale, et plus préférentiellement encore entre 5 et 30% en poids environ.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 3 à 12 et de préférence de 5 à 11, et pouvant être ajusté au moyen d'agents d'alcalinisation ou d'agents d'acidification ou de tampons antérieurement bien connus.
Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 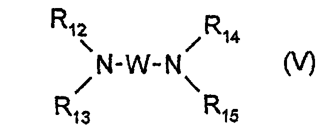 dans laquelle, W est un reste propylène éventuellement substitué par un groupement hydroxyle ou un radical alkyle en C₁-C₄ ; R₁₂ R₁₃, R₁₄ et R₁₅, simultanément ou indépendamment l'un de l'autre, représentent un atome d'hydrogène, un radical alkyle en C₁-C₆ ou hydroxyalkyle en C₁-C₆.
Les agents acidifiants sont classiquement des acides minéraux ou organiques comme par exemple les acides chlorhydrique, tartrique, citrique et phosphorique. Parmi les tampons, on peut citer par exemple, le phosphate diacide de potassium/hydroxyde de sodium.

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Un autre objet de la présente invention porte sur un procédé de teinture des fibres kératiniques, en particulier des fibres kératiniques humaines telles que les cheveux, par coloration directe, consistant à laisser agir une composition tinctoriale renfermant au moins une aminoanthraquinone cationique de formule (I) sur les fibres kératiniques sèches ou humides. On peut utiliser la composition selon l'invention en tant que composition non rincée, c'est-à-dire qu'après application de la composition sur les fibres, on seche sans rinçage intermédiaire.
Dans les autres modes d'application, on laisse agir la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLES DE PREPARATION

### EXEMPLE 1 : Préparation du bromure de 1-[2-(9,10-dioxo-9,10-dihydro-anthracen-1-ylamino)-éthyl]-3-méthyl-3H-imidazol-1-ium, (charge délocalisée dans le cycle imidazolique).

On a mis en suspension dans 50 ml de toluène, 16,5 g (0,05 mole) de 1-(2-bromo-éthylamino)-anthraquinone (RN-3591-05-7) et 4,9 g (0,06 mole) de 1-méthyl-1H-imidazole (RN-616-47-7).
On a ensuite chauffé, sous agitation, au reflux du toluène, pendant 4 heures (ce qui correspond à la disparition de l'anthraquinone de départ en chromatographie sur couche mince); puis on a essoré le produit bouillant et on l'a lavé deux fois dans l'acétate d'éthyle.
Après séchage à 40°C sous vide, on a obtenu des cristaux rouges (19,2 g) fondant avec décomposition à 184-186°C (Kofler).

L'analyse élémentaire pour **C**_{**20**} **H**_{**18**} **N**_{**3**} **O**_{**2**} **Br** était :

| **%** | **C** | **H** | **N** | **O** | **Br** |
|---|---|---|---|---|---|
| théorie | 58,27 | 4,40 | 10,19 | 7,79 | 19,38 |
| trouvé | 58,26 | 4,42 | 10,04 | 7,85 | 19,26 |

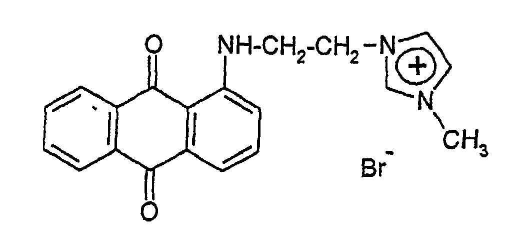

### EXEMPLE 2 : Préparation du sulfate acide de 1-méthyl-3-[3-(4-méthyl-amino-9,10-dioxo-9,10-dihydro-anthracen-1-ylamino)-propyl]-3H-imidazol-1-ium, (charge délocalisée dans le cycle imidazolique).

Dans une première étape, on a préparé la 1-(3-imidazol-yl-propylamino)-4-méthylamino-anthraquinone, en chauffant durant 9 heures, au bain-marie bouillant, et sous agitation, le mélange comprenant 47,4 g (0,15 mole) de 1-bromo-4-méthylamino-anthraquinone (RN 128-93-8), 56,3 g (0,45 mole) de 3-imidazol-1-yl-propylamine (RN 5036-48-6), et 3,0 g de sulfate de cuivre pentahydraté, dans 60 ml de 1-méthyl-2-pyrrolidone.
Puis on a versé le mélange sur 300 g d'eau glacée, essoré le précipité cristallisé ainsi obtenu, réempâté le précipité dans l'eau, et séché à 40°C sous vide sur anhydride phosphorique.
On a obtenu 45,1 g de cristaux bleu-foncé, qui, après purification par recristallisation du 1,2-diméthoxyéthane bouillant, fondaient à 140°C (Kofler).

L'analyse élémentaire pour **C**_{**21**} **H**_{**20**} **N**_{**4**} **O**_{**2**} était :

| %, | **C** | **H** | **N** | **O** |
|---|---|---|---|---|
| théorie | 69,98 | 5,59 | 15,54 | 8,88 |
| trouvé | 69,85 | 5,64 | 15,60 | 8,99 |

Dans une seconde étape, et pour quatemiser la 1-(3-imidazol-yl-propylamino)-4-méthylamino-anthraquinone obtenue à la première étape, on a mis en suspension dans 25 ml de chlorobenzène, 4,7 g (0,013 mole) de 1-(3-imidazolyl-propylamino)-4-méthylamino-anthraquinone, 1,37 ml (0,0143 mole) de sulfate de diméthyle, et on a chauffé, sous agitation, pendant 3 heures à 45°C.
On a ensuite décanté l'huile bleue en suspension obtenue, qu'on a lavée plusieurs fois dans l'acétate d'éthyle, puis séchée sous vide à 50°C.
On a obtenu 3,8 g d'un composé bleu-foncé qui a cristallisé après purification et dont le point de fusion (Kofler) était 192-194°C.
La structure était conforme en RMN ¹H et ¹³C.

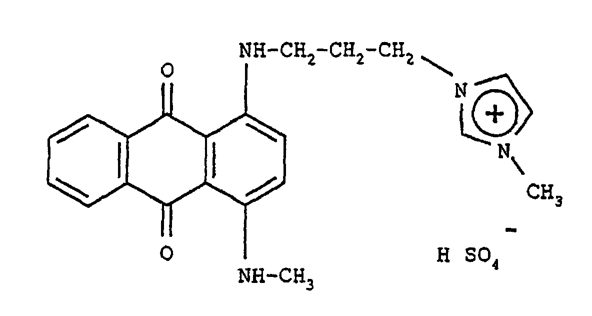

### EXEMPLES DE COMPOSITIONS TINCTORIALES

### EXEMPLE 3:

On a préparé la composition de teinture suivante

| | |
|---|---|
| Aminoanthraquinone préparée à l'exemple 1.(10⁻³ mole) | 0,412g |
| Alcool benzylique | 4,0 g |
| Polyéthylèneglycol 60E | 6,0 g |
| Hydroxyéthylcellulose | 0,7 g |
| Alkylpolyglucoside en solution aqueuse à 60% M.A* | 4,5 g M.A |
| Tampon phosphate q.s pH | 7 |
| Eau déminéralisée q.s.p. | 100, g |

| | |
|---|---|
| * Matière Active | |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance rouge-cuivré.

### EXEMPLE 4 :

On a préparé la composition de teinture suivante :

| | |
|---|---|
| Aminoanthraquinone préparée à l'exemple 2 | 0,49 g |
| Diéthanolamide oléïque | 3 g |
| Acide laurique | 1 g |
| Monoéthyléther de l'éthylèneglycol | 5 g |
| Hydroxyéthylcellulose | 2 g |
| 2-amino-2-méthyl-1-propanol q.s. pH | 9,5 |
| Eau déminéralisée q.s.p. | 100 g |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance bleu-puissant.

### EXEMPLES 5 et 6 COMPARATIFS :

On a réalisé, parallèlement à la composition de l'exemple 3, deux compositions comparatives (3A) et (3B), qui, en remplacement de l'aminoanthraquinone cationique de l'invention contenaient, en équivalent molaire, deux aminoanthraquinones cationiques de l'art antérieur décrites dans le brevet FR-1 379 649, et dans lesquelles la charge cationique était localisée sur l'azote :

### Composition comparative (3A) :

avec:

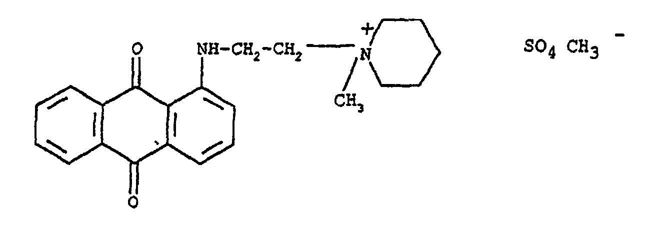

### Composition comparative (3B) :

avec :

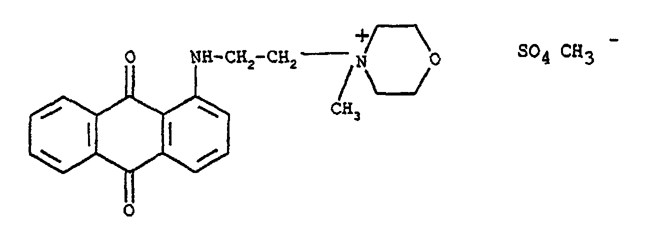

Les autres constituants des compositions comparatives (3A) et (3B) étaient identiques, en nature et en concentration, à ceux de la composition 3 conforme à l'invention.
Puis on a teint des mèches de cheveux avec chacune des compositions comparatives (3A) et (3B) suivant le même protocole et avec la même qualité de cheveux qu'à l'exemple 3.
Les nuances obtenues à l'aide de chacune des trois compositions et la nuance des cheveux non teints (composition témoin) ont été mesurées au colorimètre Minolta CM 2002 et exprimées en H,V,C, dans la notation MUNSELL (Norme ASTM D 1535-68), H désignant la nuance ou HUE, V désignant l'intensité ou VALUE, et C la pureté ou CHROMATICITE.
Ces valeurs, sur la base de cheveux permanentés, ont été consignées dans le tableau (I) suivant :

**TABLEAU (I)**

| COMPOSITIONS | **H** | **V** | **C** |
|---|---|---|---|
| (3) | 7,5R | 3,8 | 6,3 |
| (3A) | 8,6R | 3,7 | 6,3 |
| (3B) | 8,8R | 4,1 | 6,7 |
| Témoin | 4,0Y | 5,4 | 1,4 |

On a ensuite soumis les mèches teintes à l'aide de chacune des trois compositions 3, (3A) et (3B) à une épreuve lumière (XENOTEST).

### Description du test de résistance à la lumière (Xenotest) :

Les cheveux teints sont fixés sur un support (carton ou plastique). Ces supports sont disposés sur des porte-échantillons qui tournent autour d'une lampe Xénon pendant une durée de 40 heures sous un taux d'humidité de 60% HR (Humidité Relative) et à une température de 25° C.

Les nuances des mèches ayant subi une épreuve lumière, ont alors été mesurées au colorimètre CM 2002 et ont été les suivantes [réunies dans le tableau (II) ci-après] :

**TABLEAU (II)**

| COMPOSITIONS | **H** | **V** | **C** |
|---|---|---|---|
| (3) | 9,1R | 3,9 | 5,9 |
| (3A) | 0,8YR | 4,1 | 5,5 |
| (3B) | 0,8YR | 4,2 | 5,6 |

Puis on a quantifié la variation de couleur entre les mèches teintes avant Xénotest et celles teintes après Xénotest, en utilisant l'équation de NICKERSON qui définit les'indices de variation de couleur :
I= (C/5) x 2ΔH + 6ΔV + 3ΔC (cette équation étant décrite dans la publication : «Journal of the Optical Society of America», 1944-sept-Vol34-n°9-p550-570),
et on a évalué le pourcentage de dégradation des nuances, engendré par l'épreuve lumière, selon l'équation I_{b} / Iₐ x100,
I_{b} définissant l'indice de variation de couleur entre les mèches teintes après Xénotest et les mèches teintes avant Xénotest), Iₐ définissant l'indice de variation de couleur entre les mèches teintes avant Xénotest et les mèches témoin).
Ainsi, pour chacune des trois nuances obtenues avec les compositions 3, (3A) et (3B), les pourcentages de dégradation ont été les suivants :
- avec la composition 3 17,4%
- avec la composition (3A) 30,9%
- avec la composition (3B) 28,8%.

### CONCLUSION :

La composition de teinture directe comprenant une aminoanthraquinone selon la présente invention (composition 3) engendre une nuance plus résistante à la lumière que celles obtenues avec les deux compositions de teinture directe comprenant des aminoanthraquinones de l'art antérieur [compositions (3A) et (3B)], seulement 17,4% de dégradation pour la composition selon la présente invention contre 30,9% et 28,8% pour les deux compositions de l'art antérieur.

## Revendications

1. Aminoanthraquinones cationiques de formule (I) et leurs sels d'addition avec un acide 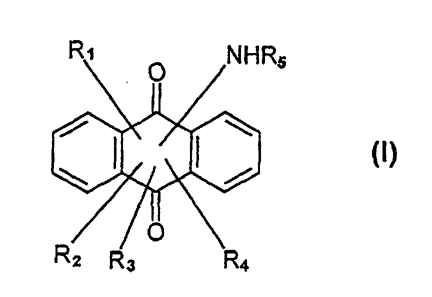 formule dans laquelle :
• **R**_{**1**}**, R**_{**2**}**, R**_{**3**} et **R**_{**4**}**,** qui peuvent être identiques ou différents, représentent un atome d'hydrogène ; un atome d'halogène ; un groupement Z défini ci-après ; un radical alkyl(C₁-C₆) ; un radical monohydroxyalkyle en C₁-C₆ ; un radical polyhydroxyalkyle en C₂-C₆ ; un radical cyano ; un radical nitro ; un radical carboxy ; un radical carbamyle ; un radical sulfo ; un radical amino substitué ou non substitué, ayant les mêmes significations que NHR₅ défini ci-dessous, et pouvant être identique ou différent ; un groupement OR₆ ou SR₆ défini ci-après.
• **R**_{**5**} désigne un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₈ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z (définis ci-après), ou pouvant former ensemble, avec l'atome d'azote auquel ils sont rattachés, un cycle à 5 ou 6 chaînons, carboné, ou contenant un ou plusieurs hétéroatomes ;
• **R**_{**6**} désigne un atome d'hydrogène ; un radical alkyle en C₁-C₆; un radical monohydroxy-alkyle en C₁-C₆ ; un radical polyhydroxyalkyle en C₂-C₆ ; un groupement Z (défini c-après) ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle en C₁-C₆ ; un radical alkyl(C₁-C₆)sulfinyl-alkyle en C₁-C₆ ; un radical alkyl(C₁-C₆)sulfonylalkyle en C₁-C₆ ; un radical alkyl(C₁-C₆)-carbonylalkyle en C₁-C₆ ; un radical aminoalkyle en (C₁-C₆) dont l'alkyle est substitué ou non substitué par un ou plusieurs radicaux hydroxy ; un radical aminoalkyle en (C₁-C₆) dont l'alkyle est substitué ou non substitué par un ou plusieurs radicaux hydroxy et dont l'aminé est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z (définis ci-après), ou pouvant former ensemble, avec l'atome d'azote auquel ils sont rattachés, un cycle à 5 ou 6 chaînons, carboné ou contenant un ou plusieurs hétéroatomes ;
• **Z** est choisi parmi les groupements cationiques insaturés de formules (II) et (III) suivantes, et les groupements cationiques saturés de formule (IV) suivante : 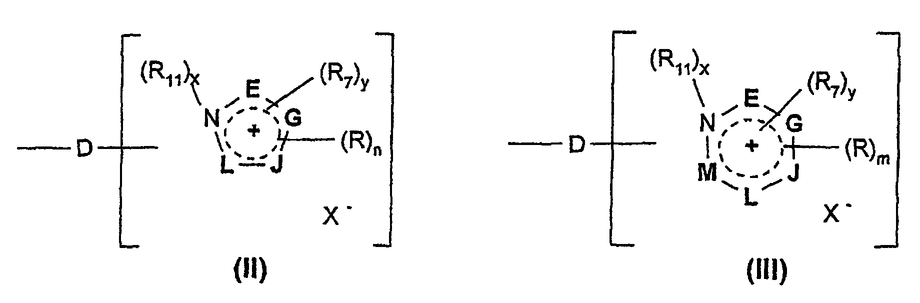 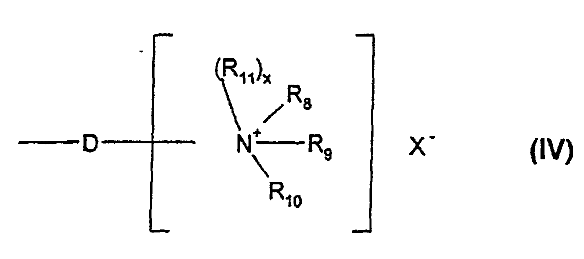 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
• R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'aminé est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₈, R₉ et R₁₀ peut également représenter un second groupement Z, identique ou différent du premier groupement Z ;
• R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'aminé est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (III):
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
- dans les groupements cationiques de formule (IV) :
- lorsque x = 0, alors le bras de liaison D est rattaché à l'atome d'azote portant les radicaux R₈ à R₁₀,
- lorsque x = 1, alors deux des radicaux R₈ à R₁₀ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
X⁻ représente un anion monovalent ou divalent,
**étant entendu que :**
le nombre de groupements cationiques insaturés Z de formule (II), dans lesquels au moins un des sommets E, G, J et L représente un atome d'azote, est au moins égal à 1.

2. Aminoanthraquinones cationiques selon la revendication 1, **caractérisée par le fait que** les cycles des groupements insaturés Z de formule (II) sont choisis parmi les cycles pyrrolique, imidazolique, pyrazolique, oxazolique, thiazolique et triazolique.

3. Aminoanthraquinones cationiques selon la revendication 1, **caractérisée par le fait que** les cycles des groupements insaturés Z de formule (III) sont choisis parmi les cycles pyridinique, pyrimidinique, pyrazinique, oxazinique et triazinique.

4. Aminoanthraquinones cationiques selon l'une quelconque des revendications précédentes, **caractérisée par le fait que** dans la formule (IV) deux des radicaux R₈, R₉ et R₁₀ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₈)sulfonyle.

5. Aminoanthraquinones cationiques selon l'une quelconque des revendications précédentes, **caractérisées par le fait que** X ⁻ est choisi parmi un atome d'halogène, un hydroxyde, un hydrogènesulfate, ou un alkyl(C₁-C₆)sulfate.

6. Aminoanthraquinones cationiques selon la revendication 1, choisies dans le groupe constitué par
- le bromure de 1-[2-(9,10-dioxo-9,10-dihydro-anthracen-1-ylamino)-éthyl]-3-méthyl-3H-imidazol-1-ium,
- le sulfate acide de 1-méthyl-3-[3-(4-méthylamino-9,10-dioxo-9,10-dihydro-anthracen-1-ylamino)-propyl]-3H-imidazol-1-ium,
- le bromure de 1-[2-(4-hydroxy-9,10-dioxo-9,10-dihydro-anthracen-1-ylamino)-ethyl]-3-methyl-3H-imidazol-1-ium,
- le méthosulfate de 1-[3-(9,10-dioxo-9,10-dihydro-anthracen-1-ylamino)-propyl]-3-methyl-3H-imidazol-1-ium,
- le méthosulfate de 1-[3-(4-hydroxy-9,10-dioxo-9,10-dihydro-anthracen-1-ylamino)-propyl]-3-methyl-3H-imidazol-1-ium,
- le méthosulfate de 1-{3-[4-(2-hydroxy-ethylamino)-9,10-dioxo-9,10-dihydro-anthracen-1-ylamino]-propyl}-3-methyl-3H-imidazol-1-ium,
- le méthosulfate de 1-{3-[4-(2,3-dihydroxy-propylamino)-9,10-dioxo-9,10-dihydro-anthracen-1-ylamino]-propyl}-3-methyl-3H-imidazol-1-ium,
- le diméthosulfate de 1,4-bis-[3-(9,10-dioxo-9,10-dihydro-anthracen-1,4-diylamino)-propyl]-3-methyl-3H-imidazol-1-ium,
- le bromure de 1-[2-(9,10-dioxo-9,10-dihydro-anthracen-2-ylamino)-ethyl]-3-methyl-3H-imidazol-1-ium,
- le bromure de 1-[2-(9,10-dioxo-9,10-dihydro-anthracen-2-ylamino)-ethyl]-2-methyl-3H-pyrazol-1-ium;
- le bromure de 1-[2-(9,10-dioxo-9,10-dihydro-anthracen-1-ylamino)-ethyl]-2-methyl-3H-pyrazol-1-ium,
- le diméthosulfate de 1,5-bis-[3-(9,10-dioxo-9,10-dihydro-anthracen-1,5-diylamino)-propyl]-3-methyl-3H-imidazol-1-ium,
- le diméthosulfate de 1,8-bis-[3-(9,10-dioxo-9,10-dihydro-anthracen-1,8-diylamino)-propyl]-3-methyl-3H-imidazol-1-ium,
- le bromure de 1-[2-(5,8-diamino-9,10-dioxo-9,10-dihydro-anthracen-1-ylamino)-ethyl]-3-methyl-3H-imidazol-1-ium,
- le méthosulfate de 1-[3-(5,8-diamino-9,10-dioxo-9,10-dihydro-anthracen-1-ylamino)-propyl]-3-methyl-3H-imidazol-1-ium.

7. Aminoanthraquinones cationiques selon l'une quelconque des revendications précédentes, **caractérisées par le fait que** les sels d'addition avec un acide sont choisis parmi les chlorhydrates, les bromhydrates, les sulfates, les citrates, les succinates, les tartrates, les lactates et les acétates.

8. Utilisation d'une aminoanthraquinone cationique de formule (I) définie selon l'une quelconque des revendications précédentes, à titre de colorant direct, dans des, ou pour la préparation de, compositions tinctoriales pour matières kératiniques, en particulier pour fibres kératiniques humaines telles que les cheveux.

9. Composition de teinture pour matières kératiniques, **caractérisée par le fait qu'**elle contient, dans un milieu approprié pour la teinture, une quantité efficace d'au moins une aminoanthraquinone cationique de formule (1) définie selon l'une quelconque des revendications 1 à 7.

10. Composition de teinture directe pour fibres kératiniques humaines et notamment les cheveux, **caractérisée par le fait qu'**elle contient, dans un milieu approprié pour la teinture, une quantité efficace d'au moins une aminoanthraquinone cationique de formule (I) définie selon l'une quelconque des revendications 1 à 7.

11. Composition selon les revendications 9 ou 10, **caractérisée par le fait qu'**elle a un pH compris entre 3 et 12.

12. Composition selon l'une quelconque des revendications 9 à 11, **caractérisée par le fait que** les aminoanthraquinones cationiques de formule (I) sont présentes dans une concentration allant de 0,005 à 12% en poids par rapport au poids total de la composition.

13. Composition selon la revendication 12, **caractérisée par le fait que** les aminoanthraquinones cationiques de formule (I) sont présentes dans une concentration allant de 0,05 à 6% en poids par rapport au poids total de la composition.

14. Composition selon l'une quelconque des revendications 9 à 13, **caractérisée par le fait qu'**elle contient d'autres colorants directs, choisis parmi des colorants nitrés benzéniques comme les nitrophénylènediamines, les nitrodiphénylamines, les nitroanilines, les éthers de phénols nitrés ou les nitrophénols, des nitropyridines, des colorants anthraquinoniques autres que ceux de formule (I), des colorants mono- ou di-azoïques, triarylméthaniques, aziniques, acridiniques, xanthéniques, ou des colorants métallifères.

15. Composition selon l'une quelconque des revendications 9 à 14, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 1 et 40% en poids par rapport au poids total de la composition.

16. Procédé de teinture des fibres kératiniques et en particulier des fibres kératiniques humaines telles que les cheveux, par coloration directe, **caractérisé par le fait qu'**on applique la composition tinctoriale définie selon l'une quelconque des revendications 9 à 15, sur les fibres kératiniques sèches ou humides, et qu'on sèche ces fibres sans rinçage intermédiaire.

17. Procédé de teinture des fibres kératiniques et en particulier des fibres kératiniques humaines telles que les cheveux, par coloration directe, **caractérisé par le fait qu'**on applique la composition tinctoriale définie selon l'une quelconque des revendications 9 à 15, sur les fibres kératiniques sèches ou humides, et qu'après avoir laissé agir la composition pendant 3 à 60 minutes environ, on rince les fibres, on les lave éventuellement, on les rince à nouveau puis on les sèche.

## Claims

1. Cationic aminoanthraquinones of formula (I) and their addition salts with an acid: 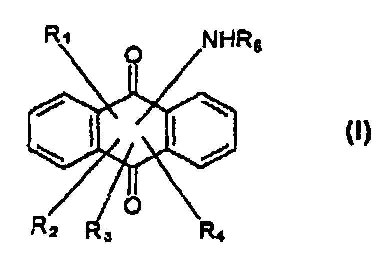 in which formula:
• **R**_{**1**}**, R**_{**2**}**, R**_{**3**} and **R**_{**4**}**,** which may be identical or different, represent a hydrogen atom; a halogen atom; a group Z defined below; a (C₁-C₆)alkyl radical; a monohydroxy(C₁-C₆ alkyl) radical; a polyhydroxy(C₂-C₆ alkyl) radical; a cyano radical; a nitro radical; a carboxyl radical; a carbamyl radical; a sulpho radical; a substituted or unsubstituted amino radical, having the same meanings as NHR₅ defined below, and which may be identical or different; a group OR₆ or SR₆ defined below;
• **R**_{**5**} designates a hydrogen atom; a group Z defined below; a C₁-C₆ alkyl radical; a monohydroxy(C₁-C₆ alkyl) radical; a polyhydroxy-(C₂-C₆ alkyl) radical; a (C₁-C₆) alkoxy(C₁-C₆ alkyl) radical; an aryl radical; a benzyl radical; a cyano(C₁-C₆ alkyl) radical; a carbamyl (C₁-C₆ alkyl) radical; an N-(C₁-C₆)-alkylcarbamyl (C₁-C₆ alkyl) radical; an N,N-di(C₁-C₆)alkylcarbamyl(C₁-C₆ alkyl) radical; a thiocarbamyl(C₁-C₆ alkyl) radical; a trifluoro(C₁-C₆ alkyl) radical; a sulpho(C₁-C₆ alkyl) radical; a (C₁-C₆)alkylcarboxy(C₁-C₆ alkyl) radical; a (C₁-C₆)alkylsulphinyl (C₁-C₆ alkyl) radical; an aminosulphonyl(C₁-C₆ alkyl) radical; an N-Z-aminosulphonyl(C₁-C₆ alkyl) radical; an N-(C₁-C₆)alkylaminosulphonyl (C₁-C₆ alkyl) radical; an N,N-di(C₁-C₆)alkylaminosulphonyl(C₁-C₆ alkyl) radical; a (C₁-C₆)alkylcarbonyl (C₁-C₆ alkyl) radical; an amino (C₁-C₆ alkyl) radical whose alkyl is substituted or unsubstituted with one or more hydroxyl radicals; an amino(C₁-C₆ alkyl) radical whose alkyl is substituted or unsubstituted with one or more hydroxyl radicals and whose amine is substituted with one or two radicals, which are identical or different, chosen from the C₁-C₆ alkyl, monohydroxy (C₁-C₆ alkyl), polyhydroxy-(C₂-C₆ alkyl), (C₁-C₆)alkylcarbonyl, carbamyl, N- (C₁-C₆) alkylcarbamyl or N, N-di (C₁-C₆) alkylcarbamyl, (C₁-C₆)alkylsulphonyl, formyl, trifluoro (C₁-C₆) alkylcarbonyl, (C₁-C₆) alkylcarboxyl or thiocarbamyl radicals, or from the groups Z (defined below), or which may form together with the nitrogen atom to which they are attached a 5- or 6-membered ring containing carbon or containing one or more heteroatoms;
• **R**_{**6**} designates a hydrogen atom; a C₁-C₆ alkyl radical; a monohydroxy(C₁-C₆ alkyl) radical; a polyhydroxy(C₂-C₆ alkyl) radical; a group Z (defined below); a (C₁-C₆) alkoxy (C₁-C₆ alkyl) radical; an aryl radical; a benzyl radical; a carboxy(C₁-C₆ alkyl) radical; a (C₁-C₆)alkylcarboxy(C₁-C₆ alkyl) radical; a cyano(C₁-C₆ alkyl) radical; a carbamyl(C₁-C₆ alkyl) radical; an N-(C₁-C₆) alkylcarbamyl(C₁-C₆ alkyl) radical; an N, N-di (C₁-C₆) alkylcarbamyl (C₁-C₆ alkyl) radical; a trifluoro(C₁-C₆ alkyl) an aminosulphonyl(C₁-C₆ alkyl) radical; an N-Z-aminosulphonyl(C₁-C₆ alkyl) radical; an N-(C₁-C₆)-alkylaminosulphonyl(C₁-C₆ alkyl) radical; an N,N-di(C₁-C₆)alkylaminosulphonyl (C₁-C₆ alkyl) radical; a (C₁-C₆)alkylsulphinyl(C₁-C₆ alkyl) radical; a (C₁-C₆ alkylsulphonyl(C₁-C₆ alkyl) radical; a (C₁-C₆)alkylcarbonyl(C₁-C₆ alkyl) radical; an amino(C₁-C₆ alkyl) radical whose alkyl is substituted or unsubstituted with one or more hydroxyl radicals; an amino(C₁-C₆ alkyl) radical whose alkyl is substituted or unsubstituted with one or more hydroxyl radicals and whose amine is substituted with one or two radicals, which are identical or different, chosen from the C₁-C₆ alkyl, monohydroxy (C₁-C₆ alkyl), polyhydroxy(C₂-C₆ alkyl), (C₁-C₆) alkylcarbonyl, formyl, trifluoro (C₁-C₆) alkylcarbonyl, (C₁-C₆)alkylcarboxyl, carbamyl, N- (C₁-C₆) alkylcarbamyl, N,N-di (C₁-C₆) alkylcarbamyl, thiocarbamyl or (C₁-C₆)alkylsulphonyl radicals, or from the groups Z (defined below), or which may form together with the nitrogen atom to which they are attached a 5- or 6-membered ring containing carbon or containing one or more heteroatoms;
• **Z** is chosen from the unsaturated cationic groups of the following formulae (II) and (III), and the saturated cationic groups of the following formula (IV) : 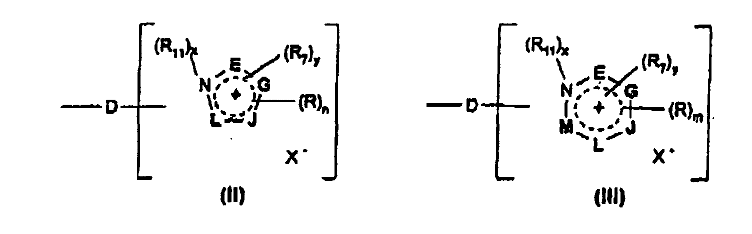 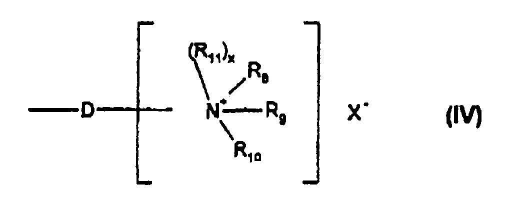 in which:
• D is a linking arm which represents a linear or branched alkyl chain preferably containing from 1 to 14 carbon atoms, which may be interrupted by one or more heteroatoms such as oxygen, sulphur or nitrogen atoms, and which may be substituted with one or more hydroxyl or C₁-C₆ alkoxy radicals, and which may carry one or more ketone functions;
• the members E, G, J, L and M, which are identical or different, represent a carbon, oxygen, sulphur or nitrogen atom;
• n is an integer between 0 and 4 inclusive;
• m is an integer between 0 and 5 inclusive;
• the radicals R, which are identical or different, represent a second group Z, which is identical or different from the first group Z, a halogen atom, a hydroxyl radical, a C₁-C₆ alkyl radical, a monohydroxy(C₁-C₆ alkyl) radical, a polyhydroxy(C₂-C₆ alkyl) radical, a nitro radical, a cyano radical, a cyano (C₁-C₆ alkyl) radical, a C₁-C₆ alkoxy radical, a tri(C₁-C₆)alkylsilane(C₁-C₆ alkyl) radical, an amido radical, an aldehydo radical, a carboxyl radical, a C₁-C₆ alkylcarbonyl radical, a thio radical, a thio (C₁-C₆ alkyl) radical, a (C₁-C₆) alkylthio radical, an amino radical, an amino radical protected with a (C₁-C₆)alkylcarbonyl, carbamyl or (C₁-C₆)alkylsulphonyl radical; a group NHR" or NR"R''' in which R" and R''', which are identical or different, represent a C₁-C₆ alkyl radical, a monohydroxy(C₁-C₆ alkyl) radical or a polyhydroxy (C₂-C₆ alkyl) radical;
• R₇ represents a C₁-C₆ alkyl radical, a monohydroxy(C₁-C₆ alkyl) radical, a polyhydroxy (C₂-C₆ alkyl) radical, a cyano (C₁-C₆ alkyl) radical, a tri (C₁-C₆) alkylsilane (C₁-C₆ alkyl) radical, a (C₁-C₆) alkoxy(C₁-C₆ alkyl) radical, a carbamyl-(C₁-C₆ alkyl) radical, a (C₁-C₆)alkylcarboxy-(C₁-C₆ alkyl) radical, a benzyl radical, a second group Z which is identical or different from the first group Z;
• R₈, R₉ and R₁₀, which are identical or different, represent a C₁-C₆ alkyl radical, a monohydroxy-(C₁-C₆ alkyl) radical, a polyhydroxy(C₂-C₆ alkyl) radical, a (C₁-C₆)alkoxy(C₁-C₆ alkyl) radical, a cyano(C₁-C₆ alkyl) radical, an aryl radical, a benzyl radical, an amido(C₁-C₆ alkyl) radical, a tri(C₁-C₆)alkylsilane(C₁-C₆ alkyl) radical or an amino(C₁-C₆ alkyl) radical whose amine is protected with a (C₁-C₆)alkylcarbonyl, carbamyl or (C₁-C₆)alkylsulphonyl radical; two of the radicals R₈, R₉ and R₁₀ may also form together with the nitrogen atom to which they are attached a saturated 5- or 6-membered ring containing carbon or capable of containing one or more heteroatoms such as, for example, a pyrrolidine ring, a piperidine ring, a piperazine ring or a morpholine ring, it being possible for the said ring to be substituted or unsubstituted with a halogen atom, a hydroxyl radical, a C₁-C₆ alkyl radical, a monohydroxy-(C₁-C₆ alkyl) radical, a polyhydroxy(C₂-C₆ alkyl) radical, a nitro radical, a cyano radical, a cyano(C₁-C₆ alkyl) radical, a C₁-C₆ alkoxy radical, a tri(C₁-C₆) aikylsilane (C₁-C₆ alkyl) radical, an amido radical, an aldehydo radical, a carboxyl radical, a keto(C₁-C₆ alkyl) radical, a thio radical, a thio(C₁-C₆ alkyl) radical, a (C₁-C₆)alkylthio radical, an amino radical, an amino radical protected with a (C₁-C₆)alkylcarbonyl, carbamyl or (C₁-C₆)alkylsulphonyl radical;
one of the radicals R₈, R₉ and R₁₀ may also represent a second group Z, which is identical or different from the first group Z;
• R₁₁ represents a C₁-C₆ alkyl radical; a monohydroxy (C₁-C₆ alkyl) radical; a polyhydroxy-(C₂-C₆ alkyl) radical; an aryl radical; a benzyl radical; an amino(C₁-C₆ alkyl) radical, an amino(C₁-C₆ alkyl) radical whose amine is protected with a (C₁-C₆)alkylcarbonyl, carbamyl or (C₁-C₆)alkylsulphonyl radical; a carboxy(C₁-C₆ alkyl) radical; a cyano(C₁-C₆ alkyl) radical; a carbamyl(C₁-C₆ alkyl) radical; a trifluoro(C₁-C₆ alkyl) radical; a tri(C₁-C₆)alkylsilane (C₁-C₆ alkyl) radical; a sulphonamido(C₁-C₆ alkyl) radical; a (C₁-C₆)alkylcarboxy(C₁-C₆ alkyl) radical; a (C₁-C₆)alkylsulphinyl(C₁-C₆ alkyl) radical; a (C₁-C₆)alkylsulphonyl(C₁-C₆ alkyl) radical; a (C₁-C₆)alkylketo(C₁-C₆ alkyl) radical; an N-(C₁-C₆)alkylcarbamyl(C₁-C₆ alkyl) radical; an N-(C₁-C₆)alkylsulphonamido(C₁-C₆ alkyl) radical;
• x and y are integers equal to 0 or 1; with the following conditions:
- in the unsaturated cationic groups of formula (II)
- when x = 0, the linking arm D is attached to the nitrogen atom,
- when x = 1, the linking arm D is attached to one of the members E, G, J or L,
- y may only take the value 1:
1) when the members E, G, J and L simultaneously represent a carbon atom, and the radical R₇ is carried by the nitrogen atom of the unsaturated ring; or alternatively
2) when at least one of the members E, G, J and L represents a nitrogen atom onto which the radical R₇ is attached;
- in the unsaturated cationic groups of formula (III) :
- when x = 0, the linking arm D is attached to the nitrogen atom,
- when x = 1, the linking arm D is attached to one of the members E, G, J, L or M,
- y may only take the value 1 when at least one of the members E, G, J, L and M represents a divalent atom, and the radical R₇ is carried by the nitrogen atom of the unsaturated ring;
- in the cationic groups of formula (IV):
- when x = 0, then the linking arm D is attached to the nitrogen atom carrying the radicals R₈ to R₁₀,
- when x = 1, then two of the radicals R₈ to R₁₀ conjointly form with the nitrogen atom to which they are attached a saturated 5- or 6-membered ring as defined above; and the linking arm D is carried by a carbon atom of the said saturated ring;
• **X**⁻ represents a monovalent or divalent anion,
**it being understood that:**
the number of unsaturated cationic groups Z of formula (II), in which at least one of the members E, G, J and L represents a nitrogen atom, is at least equal to 1.

2. Cationic aminoanthraquinones according to Claim 1, **characterized in that** the rings of the unsaturated groups Z of formula (II) are chosen from the pyrrole, imidazole, pyrazole, oxazole, thiazole and triazole rings.

3. Cationic aminoanthraquinones according to Claim 1, **characterized in that** the rings of the unsaturated groups Z of formula (III) are chosen from the pyridine, pyrimidine, pyrazine, oxazine and triazine rings.

4. Cationic aminoanthraquinones according to any one of the preceding claims, **characterized in that** in formula (IV), two of the radicals R₈, R₉ and R₁₀ form a pyrrolidine ring, a piperidine ring, a piperazine ring or a morpholine ring; it being possible for the said ring to be substituted or unsubstituted with a halogen atom, a hydroxyl radical, a C₁-C₆ alkyl radical, a monohydroxy(C₁-C₆ alkyl) radical, a polyhydroxy(C₂-C₆ alkyl) radical, a nitro radical, a cyano radical, a cyano(C₁-C₆ alkyl) radical, a C₁-C₆ alkoxy radical, a tri(C₁-C₆)alkylsilane(C₁-C₆ alkyl) radical, an amido radical, an aldehydo radical, a carboxyl radical, a (C₁-C₆ alkyl)carbonyl radical, a thio radical, a thin (C₁-C₆ alkyl) radical, a (C₁-C₆)alkylthio radical, an amino radical, an amino radical protected with a (C₁-C₆)alkylcarbonyl, carbamyl or (C₁-C₆)alkylsulphonyl radical.

5. Cationic aminoanthraquinones according to any one of the preceding claims, **characterized in that** X⁻ is chosen from a halogen atom, a hydroxide, a hydrogen sulphate or a (C₁-C₆) alkyl sulphate.

6. Cationic aminoanthraquinones according to Claim 1, chosen from the group consisting of:
- 1-[2-(9,10-Dioxo-9,10-dihydroanthracen-1-ylamino)ethyl]-3-methyl-3H-imidazol-1-ium bromide,
- 1-Methyl-3-[3-(4-methylamino-9,10-dioxo-9,10-dihydroanthracen-1-ylamino)propyl]-3H-imidazol-1-ium hydrogen sulphate,
- 1-[2-(4-Hydroxy-9,10-dioxo-9,10-dihydro-anthracen-1-ylamino)ethyl]-3-methyl-3H-imidazol-1-ium bromide,
- 1-[3-(9,10-Dioxo-9,10-dihydroanthracen-1-ylamino)propyl]-3-methyl-3H-imidazel-1-ium methosulphate,
- 1-[3-(4-Hydroxy-9,10-dioxo-9,10-dihydro-anthracen-1-ylamino)propyl-]-3-methyl-3H-imidazol-1-ium methosulphate,
- 1-[3-[4-(2-Hydroxyethylamino)-9,10-dioxo-9,10-dihydroanthracen-l-ylamino]propyl}-3-methyl-3H-imidazol-1-ium methosulphate,
- 1-{3-[4-(2,3-Dihydroxypropylamino)-9,10-dioxo-9,10-dihydroanthracen-1-ylamino]propyl}-3-methyl-3H-imidazol-1-ium methosulphate,
- 1,4-bis[3-(9,10-Dioxo-9,10-dihydroanthracen-1,4-diylamino)propyl]-3-methyl-3H-imidazol-1-ium dimethosulphate,
- 1-[2-(9,10-Dioxo-9,10-dihydroanthracen-2-ylamino)ethyl]-3-methyl-3H-imidazol-1-ium bromide,
- 1-[2-(9,10-Dioxo-9,10-dihydroanthracen-2-ylamino)ethyl]-2-methyl-3H-pyrazol-1-ium bromide,
- 1-[2-(9,10-Dioxo-9,10-dihydroanthracen-1-ylamino)ethyl] -2-methyl-3H-pyrazol-1-ium bromide,
- 1,5-bis [3-(9,10-Dioxo-9,10-dihydroanthracen-1,5-diylamino)propyl}-3-methyl-3H-imidazol-1-ium dimethosulphate,
- 1,8-bis[3-(9,10-Dioxo-9,10-dihydroanthracen-1,8-diylamino)propyl]-3-methyl-3H-imidazol-1-ium dimethosulphate,
- 1-[2-(5,8-Diamino-9,10-dioxo-9,10-dihydro-anthracen-1-ylamino)ethyl]-3-methyl-3H-imidazol-1-ium bromide,
- 1-[3-(5,8-Diamino-9, 10-dioxo-9,10-dihydro-anthracen-1-ylamino)propyl]-3-methyl-3H-imidazol-1-ium methosulphate.

7. Cationic aminoanthraquinones according to any one of the preceding claims, **characterized in that** the addition salts with an acid are chosen from hydrochlorides, hydrobromides, sulphates, citrates, succinates, tartrates, lactates and acetates.

8. Use of a cationic aminoanthraquinone of formula (I) defined according to any one of the preceding claims, as direct dye, in, or for the preparation of, dyeing compositions for keratinous materials, in particular for human keratinous fibres such as hair.

9. Dyeing composition for keratinous materials, **characterized in that** it contains, in a medium appropriate for dyeing, an effective quantity of at least one cationic aminoanthraquinone of formula (I) defined in any one of Claims 1 to 7.

10. Direct dyeing composition for human keratinous fibres, and in particular hair, **characterized in that** it contains, in a medium appropriate for dyeing, an effective quantity of at least one cationic aminoanthraquinone of formula (I) defined in any one of Claims 1 to 7.

11. Composition according to Claim 9 or 10, **characterized in that** it has a pH of between 3 and 12.

12. Composition according to any one of Claims 9 to 11, **characterized in that** the cationic aminoanthraquinones of formula (I) are present in a concentration ranging from 0.005 to 12% by weight relative to the total weight of the composition.

13. Composition according to Claim 12, **characterized in that** the cationic aminoanthraquinones of formula (I) are present in a concentration ranging from 0.05 to 6% by weight relative to the total weight of the composition.

14. Composition according to any one of Claims 9 to 13, **characterized in that** it contains other direct dyes, chosen from nitrobenzene dyes such as nitrophenylenediamines, nitrodiphenylamines, nitroanilines, nitrophenol ethers or nitrophenols, nitropyridines, anthraquinone dyes different from those of formula (I), mono- or diazo, triarylmethane, azine, acridine and xanthene dyes, or metal-containing dyes.

15. Composition according to any one of Claims 9 to 14, **characterized in that** the medium appropriate for dyeing is an aqueous medium consisting of water and/or organic solvents chosen from alcohols, glycols and glycol ethers, in proportions of between 1 and 40% by weight relative;to the total weight of the composition.

16. Method of dyeing keratinous fibres and in particular human keratinous fibres such as hair by direct dyeing, **characterized in that** the dyeing composition defined in any one of Claims 9 to 15 is applied to the dry or wet keratinous fibres, and **in that** these fibres are dried without intermediate rinsing.

17. Method of dyeing keratinous fibres and in particular human keratinous fibres such as hair by direct dyeing, **characterized in that** the dyeing composition defined in any one of Claims 9 to 15 is applied to the dry or wet keratinous fibres, and **in that** after allowing the composition to act for 3 to 60 minutes approximately, the fibres are rinsed, they are optionally washed, they are rinsed again and then they are dried.

## Patentansprüche

1. Kationische Aminoanthrachinone der folgenden Formel (I) und ihre Additionssalze mit einer Säure: 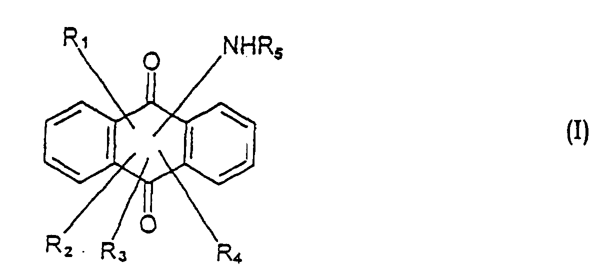 in der Formel:
• R₁, R₂, R₃ und R₄, die identisch oder voneinander verschieden sein können, bedeuten Wasserstoff; Halogen; eine nachfolgend definierte Gruppe Z; C₁₋₆-Alkyl; C₁₋₆-Monohydroxyalkyl; C₂₋₆-Polyhydroxyalkyl; Cyano; Nitro; Carboxy; Carbamoyl; Sulfo; eine substituierte oder unsubstituierte Aminögruppe, die die nachstehend angegebenen Bedeutungen der Gruppe NHR₅ aufweist und identisch oder verschieden sein kann; eine nachfolgend definierte Gruppe OR₆ oder SR₆;
• R₅ bedeutet Wasserstoff; eine nachfolgend definierte Gruppe Z; C₁₋₆-Alkyl; C₁₋₆-Monohydroxyalkyl; C₂₋₆-Polyhydroxyalkyl; Alkoxy-(C₁₋₆)alkyl(C₁₋₆); Aryl; Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋ ₆); C₁₋₆-Aminosulfonylalkyl; N-Z-C₁₋₆-Aminosulfonylalkyl; N-Alkyl(C₁₋₆)aminosulfonylalkyl(C₁₋₆); N,N-Dialkyl(C₁₋₆)-aminosulfonylalkyl(C₁₋₆); Alkyl(C₁₋₆)carbonylalkyl(C₁₋₆); C₁₋₆-Aminoalkyl, wobei die Alkylgruppe unsubstituiert vorliegt oder mit einer oder mehreren Hydroxygruppen substituiert ist; C₁₋₆-Aminoalkyl, wobei die Alkylgruppe unsubstituiert vorliegt oder mit einer oder mehreren Hydroxygruppen substituiert ist und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl-(C₁₋₆)carbonyl, Carbamoyl, N-Alkyl(C₁₋₆)-carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl oder den Gruppen Z (nachstehend definiert); oder die gemeinsam mit dem Stickstoffatom, an das sie gebunden sind, einen 5- oder 6-gliedrigen Ring bilden können, der nur Kohlenstoffatome aufweist oder auch ein oder mehrere Heteroatome enthält;
• R₆ bedeutet Wasserstoff; C₁₋₆-Alkyl; C₁₋₆-Monohydroxyalkyl; C₂₋₆-Polyhydroxyalkyl; eine Gruppe Z (nachstehend definiert); Alkoxy-(C₁₋₆)alkyl-(C₁₋₆); Aryl; Benzyl; C₁₋₆-Carboxyalkyl; Alkyl(C₁₋₆)-carboxyalkyl(C₁₋₆); C₁₋₆-Cyanoalkyl; C₁₋₆-Carbamoylalkyl; N-Alkyl-(C₁₋₆)carbamoylalkyl(C₁₋₆); N,N-Dialkyl(C₁₋₆)carbamoylalkyl(C₁₋₆); C₁₋₆-Trifluoralkyl; C₁₋₆-Aminosulfonylalkyl; N-Z-C₁₋₆-Aminosulfonylalkyl; N-Alkyl(C₁₋₆)aminosulfonylalkyl(C₁₋₆); N,N-Dialkyl-(C₁₋₆)aminosulfonylalkyl(C₁₋₆); Alkyl(C₁₋₆)sulfinylalkyl(C₁₋₆); Alkyl-(C₁₋₆)sulfonylalkyl(C₁₋₆); Alkyl- (C₁₋₆)carbonylalkyl(C₁₋₆); C₁₋₆-Aminoalkyl, wobei die Alkylgruppe unsubstituiert vorliegt oder mit einer oder mehreren Hydroxygruppen substituiert ist; C₁₋₆-Aminoalkyl, wobei die Alkylgruppe unsubstituiert vorliegt oder mit einer oder mehreren Hydroxygruppen substituiert ist und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)-carbonyl, Alkyl(C₁₋₆)-carboxy, Carbamoyl, N-Alkyl(C₁₋₆)carbamoyl, N,N-Dialkyl(C₁₋₆)carbamoyl, Thiocarbamoyl, Alkyl(C₁₋₆)sulfonyl oder den Gruppen Z; oder die gemeinsam mit dem Stickstoffatom, an das sie gebunden sind, einen 5- oder 6-gliedrigen Ring bilden können, der nur Kohlenstoffatome enthält oder auch ein oder mehrere Heteroatome aufweist;
• Z ist unter den ungesättigten kationischen Gruppen der folgenden Formeln (II) und (III) und den gesättigten kationischen Gruppen der folgenden Formel (IV) ausgewählt: 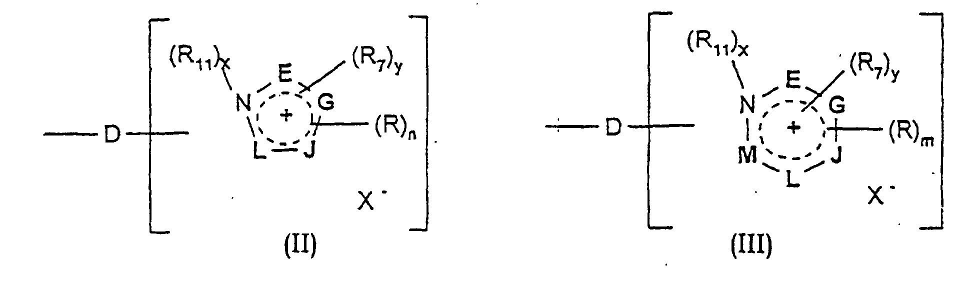 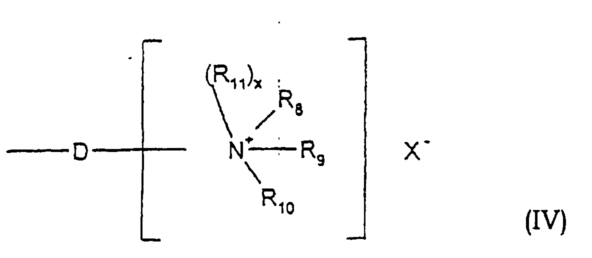 worin bedeuten:
• D eine Verbindungsgruppe, die eine Alkylkette mit vorzugsweise 1 bis 14 Kohlenstoffatome bedeutet, die geradkettig oder verzweigt vorliegt und mit einem oder mehreren Heteroatomen, wie beispielsweise Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketogruppen tragen kann;
• E, G, J, L und M, die identisch oder voneinander verschieden sind, bedeuten ein Kohlenstoffatom, ein Sauerstoffatom, ein Schwefelatom oder ein Stickstoffatom;
• n ist 0 öder eine ganze Zahl von 1 bis 4;
• m ist 0 oder eine ganze Zahl von 1 bis 5;
• die Gruppen R, die identisch oder voneinander verschieden sind, bedeuten eine zweite Gruppe Z, die identisch mit der ersten Gruppe Z oder von ihr verschieden sein kann, Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)-silanalkyl(C₁₋₆), Amido, Aldehydo, Carboxy, C₁₋₆-Alkylcarbonyl, Thio, C₁₋₆-Thioalkyl, Alkyl(C₁₋₆)thio, Amino, eine Aminogruppe, die mit Alkyl(C₁₋₆)carbonyl, Carbamoyl oder Alkyl(C₁₋₆)sulfonyl geschützt ist; eine Gruppe NHR" oder NR"R"', worin R" und R"', die identisch oder voneinander verschieden sind, C₁₋₆-Alkyl, C₁₋ ₆-Monohydroxyalkyl oder C₂₋₆-Polyhydroxyalkyl bedeuten;
• R₇ bedeutet C₁₋₆-Alkyl, C₁₋₆-Monohydroxyalkyl, C₂₋₆-Polyhydroxyalkyl, C₁₋₆-Cyanoalkyl, Trialkyl(C₁₋₆)silanalkyl(C₁₋₆), Alkoxy(C₁₋₆)alkyl(C₁₋₆), C₁₋₆-Carbamoylalkyl, Alkyl(C₁₋₆)carboxyalkyl(C₁₋₆), Benzyl oder eine zweite Gruppe Z, die identisch mit der ersten Gruppe Z oder von ihr verschieden ist;
• R₈, R₉ und R₁₀, die identisch oder voneinander verschieden sind, C₁₋₆-Alkyl, C₁₋₆-Monohydroxyalkyl, C₂₋₆-Polyhydroxyalkyl, Alkoxy(C₁₋₆)alkyl(C₁₋₆), C₁₋₆-Cyanoalkyl, Aryl, Benzyl, C₁₋₆-Amidoalkyl, Trialkyl(C₁₋₆)silanalkyl(C₁₋₆) oder C₁₋₆-Aminoalkyl, wobei die Aminogruppe mit Alkyl(C₁₋₆)carbonyl, Carbamoyl oder Alkyl(C₁₋₆)sulfonyl geschützt ist; zwei der Gruppen R₈, R₉ und R₁₀ können auch gemeinsam mit dem Stickstoffatom, an das sie gebunden sind, einen gesättigten 5- oder 6-gliedrigen Ring bilden, der nur Kohlenstoffatome aufweist oder ein oder mehrere Heteroatome enthalten kann, beispielsweise Pyrrolidin, Piperidin, Piperazin oder Morpholin, wobei der Ring unsubstituiert vorliegen kann oder substituiert sein kann mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆) silanalkyl(C₁₋ ₆), Amido, Aldehydo, Carboxy, C₁₋₆-Ketoalkyl, Thio, C₁₋₆-Thioalkyl, Alkyl(C₁₋₆)thio, Amino, einer Aminogruppe, die mit Alkyl-(C₁₋₆)Carbonyl, Carbamoyl oder Alkyl(C₁₋₆)sulfonyl geschützt ist;
wobei eine der Gruppen R₈, R₉ und R₁₀ auch eine zweite Gruppe Z bedeuten kann, die identisch mit der ersten Gruppe Z oder von ihr verschieden ist;
• R₁₁ bedeutet C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)-silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N- Alkyl(C₁₋₆)carbamoylalkyl(C₁₋₆); N-Alkyl(C₁₋₆)sulfonamidoalkyl(C₁₋₆);
• x und y bedeuten 0 oder 1; mit den folgenden Bedingungen:
- in den ungesättigten kationischen Gruppen der Formel (II):
- die Verbindungsgruppe D ist an das Stickstoffatom gebunden, wenn x = 0,
- die Verbindungsgruppe D ist'an eines der Atome E, G, J oder L gebunden, wenn x = 1,
- y kann nur den Wert 1 annehmen, wenn: ,
1) die Gruppe R₇ von dem Stickstoffatom des ungesättigten Rings getragen wird, wenn die Atome E, G, J und L gleichzeitig ein Kohlenstoffatom bedeuten; oder
2) mindestens eines der Atome E, G, J und L ein Stickstoffatom bedeutet, an das R₇ gebunden ist;
- in den ungesättigten kationischen Gruppen der Formel (III):
- die Verbindungsgruppe D an das Stickstoffatom gebunden ist, wenn x = 0,
- die Verbindungsgruppe D an eines der Atome E, G, J, L oder M gebunden ist, wenn x = 1,
- y nur den Wert 1 annehmen kann, wenn mindestens eines der Atome E, G, J, L und M ein zweiwertiges Atom bedeutet und die Gruppe R₇ von dem Stickstoffatom des ungesättigten Rings getragen wird;
- in den kationischen Gruppen der Formel (IV):
- die Verbindungsgruppe D an das Stickstoffatom gebunden ist, das die Gruppen R₈ bis R₁₀ trägt, wenn x = 0,
- zwei der Gruppen R₈ bis R₁₀ gemeinsam mit dem Stickstoffatom, an das sie gebunden sind, einen oben definierten gesättigten 5- oder 6-gliedrigen Ring bilden und die Verbindungsgruppe D von einem Kohlenstoffatom des gesättigten Rings getragen wird, wenn x = 1;
• X⁻ bedeutet ein einwertiges oder zweiwertiges Anion;
mit der Maßgabe, daß:
- die Anzahl der ungesättigten kationischen Gruppen Z der Formel (II), in denen mindestens eines der Atome E,G, J und L Stickstoff bedeutet, mindestens 1 ist.

2. Kationische Aminoanthrachinone nach Anspruch 1, **dadurch gekennzeichnet, daß** die Ringe der ungesättigten Gruppen Z der Formel (II) unter Pyrrolringen, Imidazolringen, Pyrazolringen, Oxazolringen, Thiazolringen und Triazolringen ausgewählt sind.

3. Kationische Aminoanthrachinone nach Anspruch 1, **dadurch gekennzeichnet, daß** die Ringe der ungesättigten Gruppen Z der Formel (III) unter den Pyridinringen, Pyrimidinringen, Pyrazinringen, Oxazinringen und Triazinringen ausgewählt sind.

4. Kationische Aminoanthrachinone nach einem der vorhergehenden Ansprüche, **dadurch gekennzeichnet, daß** in der Formel (IV) zwei der Gruppen R₈, R₉ und R₁₀ einen Pyrrolidinring, Piperidinring, Piperazinring oder Morpholinring bilden, wobei der Ring unsubstituiert vorliegen oder gegebenenfalls mit einem Halogenatom, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋ ₆)thio, Amino oder einer Aminogruppe substituiert sein kann, die mit Alkyl(C₁₋₆)carbonyl, Carbamoyl oder Alkyl(C₁₋₆)sulfonyl geschützt ist.

5. Kationische Aminoanthrachinone nach einem der vorhergehenden Ansprüche, **dadurch gekennzeichnet, daß** X⁻ Halogen, Hydroxid, Hydrogensulfat oder Alkyl(C₁₋₆)sulfat bedeutet.

6. Verbindungen nach einem der vorhergehenden Ansprüche, **dadurch gekennzeichnet, daß** sie ausgewählt sind unter:
- 1-[2-(9,10-Dioxo-9,10-dihydro-anthracen-1-ylamino)-ethyl]-3-methyl-3H-imidazol-1-ium-bromid;
- 1-Methyl-3-[3-(4-methylamino-9,10-dioxo-9,10-dihydroanthracen-1-ylamino)-propyl]-3H-imidazol-1-ium-hydrogensulfat;
- 1-[2-(4-Hydroxy-9,10-dioxo-9,10-dihydro-anthracen-1-ylamino)-ethyl]-3-methyl-3H-imidazol-1-ium-bromid;
- 1-[3-(9,10-Dioxo-9,10-dihydro-anthracen-1-ylamino)-propyl]-3-methyl-3H-imidazol-1-ium-methosulfat;
- 1-[3-(4-Hydroxy-9,10-dioxo-9,10-dihydro-anthracen-1-ylamino)-propyl]-3-methyl-3H-imidazol-1-ium-methosulfat;
- 1-{3-[4-(2-Hydroxy-ethylamino)-9,10-dioxo-9,10-dihydroanthracen-1-ylamino)-propyl]-3-methyl-3H-imidazol-1-iummethosulfat;
- 1-{3-[4-(2,3-Dihydroxy-propylamino)-9,10-dioxo-9,10-dihydroanthracen-1-ylamino)-propyl]-3-methyl-3H-imidazol-1-iummethosulfat;
- 1,4-Bis[3-(9,10-dioxo-9,10-dihydro-anthracen-1,4-diylamino)-propyl]-3-methyl-3H-imidazol-1-ium-dimethosulfat;
- 1-[2-(9,10-Dioxo-9,10-dihydro-anthracen-2-ylamino)-ethyl]-3-methyl-3H-imidazol-1-ium-bromid;
- 1-[2-(9,10-Dioxo-9,10-dihydro-anthracen-2-ylamino)-ethyl]-2-methyl-3H-pyrazol-1-ium-bromid;
- 1-[2-(9,10-Dioxo-9,10-dihydro-anthracen-1-ylamino)-ethyl]-2-methyl-3H-pyrazol-1-ium-bromid;
- 1,5-Bis[3-(9,10-dioxo-9,10-dihydro-anthracen-1,5-diylamino)-propyl]-3-methyl-3H-imidazol-1-ium-dimethosulfat;
- 1,8-Bis[3-(9,10-dioxo-9,10-dihydro-anthracen-1,8-diylamino)-propyl]-3-methyl-3H-imidazol-1-ium-dimethosulfat;
- 1-[2-(5,8-Diamino-9,10-dioxo-9,10-dihydro-anthracen-1-ylamino)-ethyl]-3-methyl-3H-imidazol-1-ium-bromid; und
- 1-[3-(5,8-Diamino-9,10-dioxo-9,10-dihydro-anthracen-1-ylamino)-propyl]-3-methyl-3H-pyrazol-1-ium-methosulfat.

7. Kationische Aminoanthrachinone nach einem der vorhergehenden Ansprüche, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

8. Verwendung von kationischen Aminoanthrachinonen der Formel (I) nach einem der vorhergehenden Ansprüche als Direktfarbstoffe in Farbmittelzusammensetzungen oder zur Herstellung von Farbmittelzusammensetzungen zum Färben von Keratinsubstanzen und insbesondere menschlichen Keratinfasern, wie dem Haar.

9. Zusammensetzung zum Färben von Keratinsubstanzen, **dadurch gekennzeichnet, daß** sie in einem zum Färben geeigneten Medium mindestens ein kationisches Aminoanthrachinon der Formel (I) nach einem der Ansprüche 1 bis 7 in einer wirksamen Menge enthält.

10. Zusammensetzung zum direkten Färben von menschlichen Keratinfasern und insbesondere zum Färben der Haare, **dadurch gekennzeichnet, daß** sie in einem zum Färben geeigneten Medium mindestens ein kationisches Aminoanthrachinon der Formel (I) nach einem der Ansprüche 1 bis 7 in einer wirksamen Menge enthält.

11. Zusammensetzung nach den Ansprüchen 9 oder 10, **dadurch gekennzeichnet, daß** der pH-Wert im Bereich von 3 bis 12 liegt.

12. Zusammensetzung nach einem der Ansprüche 9 bis 11, **dadurch gekennzeichnet, daß** die kationischen Aminoanthrachinone der Formel (I) in einer Konzentration von 0,005 bis 12 Gew.-%, bezogen auf das Gesamtgewicht der Zusammerisetzung, vorliegen.

13. Zusammensetzung nach Anspruch 12, **dadurch gekennzeichnet, daß** die kationischen Aminoanthrachinone der Formel (I) in einer Konzentration von 0,05 bis 6 Gew.-%, bezogen auf das Gesamtgewicht der Zusammensetzung, vorliegen.

14. Zusammensetzung nach einem der Ansprüche 9 bis 13, **dadurch gekennzeichnet, daß** sie weitere Direktfarbstoffe enthält, die unter den Nitrobenzol-Farbstoffen, wie Nitrophenylendiaminen, Nitrodiphenylaminen, Nitroanilinen, Nitrophenolethern oder Nitrophenolen, Nitropyridinen, Anthrachinon-Farbstoffen, die von den Verbindungen der Formel (I) verschieden sind, Mono- oder Diazofarbstoffen, Triarylmethan-Farbstoffen, Azin-Farbstoffen, Acridin-Farbstoffen und Xanthen-Farbstoffen oder metallhaltigen Farbstoffen ausgewählt sind.

15. Zusammensetzung nach einem der Ansprüche 9 bis 14, **dadurch gekennzeichnet, daß** das zum Färben geeignete Medium ein wäßriges Medium ist, das aus Wasser und/oder organischen Lösungsmitteln in Mengenanteilen von 1 bis 40 Gew.-%, bezogen auf das Gesamtgewicht der Zusammensetzung, besteht, die unter den Alkoholen, Glykolen und Glykolethern ausgewählt sind.

16. Verfahren zum Färben von Keratinfasern und insbesondere menschlichen Keratinfasern, wie dem Haar, durch direktes Färben, **dadurch gekennzeichnet, daß** eine Farbmittelzusammensetzung nach einem der Ansprüche 9 bis 15 auf die trockenen oder feuchten Keratinfasern aufgetragen wird und die Fasern ohne zwischenzeitliches Spülen getrocknet werden.

17. Verfahren zum Färben von Keratinfasern und insbesondere menschlichen Keratinfasern, wie dem Haar, durch direktes Färben, **dadurch gekennzeichnet, daß** eine Farbmittelzusammensetzung nach einem der Ansprüche 9 bis 15 auf die trockenen oder feuchten Keratinfasern aufgetragen wird und nach einer Einwirkzeit von etwa 3 bis 60 min die Fasern gespült, gegebenenfalls gewaschen, nochmals gespült und getrocknet werden.
